# FASCICULE DE BREVET EUROPEEN

(11) **EP 2 868 313 B1**
(45) Date de publication et mention de la délivrance du brevet: **19.06.2019**
(21) Numéro de dépôt: 14190494.6
(22) Date de dépôt: 27.10.2014
(51) Int. Cl.: A61Q 19/08, A61K 8/73, A61K 8/67, A61K 31/375, A61K 31/715, A61K 31/728

(54) **Association vitamine C et acide hyaluronique pour le traitement des effets du vieillissement cutané**
Verbindung von Vitamin C und Hyaluronsäure für die Behandlung von Hautalterungseffekten
Combination of vitamin C and hyaluronic acid for the treatment of skin ageing effects

(30) Priorité: 29.10.2013 FR 1360550
(43) Date de publication de la demande: 06.05.2015
(73) Titulaire: Dermaconcept JMC, 80000 Amiens (FR)
(72) Inventeur: Humbert, Philippe, 25290 Ornans (FR); Allart, Jean-Claude, 62520 Le Touquet (FR); Lefevre, Jean-Marie, 80000 Amiens (FR); Peyrot, Jacques, 63000 Clermont-Ferrand (FR)
(74) Mandataire: Peguet, Wilfried

(56) Documents cités:
- FR-A1- 2 939 799
- JP-A- 2002 223 726
- JP-A- 2012 077 044
- US-A1- 2003 165 456
- US-A1- 2007 077 292
- DATABASE GNPD [Online] MINTEL; 30 septembre 2008 (2008-09-30), "Meso-Mask Anti-Wrinkle Lightening Mask", XP002737019, Database accession no. 964767

## Description

La présente invention se rapporte à une composition cosmétique et/ou dermatologique comprenant l'association d'acide hyaluronique de faible poids moléculaire et d'acide ascorbique, et d'un oligosaccharide d'acide glucuronique autre que l'acide hyaluronique ou un de ses sels.

Elle s'applique typiquement, mais non exclusivement, au traitement de l'atrophie cutanée sénile, et plus généralement au traitement et à la prévention des signes du vieillissement cutané.

L'atrophie cutanée sénile est une altération particulière de la fonction de protection mécanique de la peau. Plus particulièrement, cette protection peut se trouver fortement dégradée par le vieillissement de la peau résultant de facteurs chronologiques, hormonaux, médicamenteux (e.g. utilisation prolongée de corticostéroïdes topiques ou systémiques), ... etc, et aussi de l'exposition solaire.

L'atrophie cutanée sénile, parfois dénommée "dermatoporose", peut débuter après la soixantaine, mais s'observe surtout au-delà de 70 ans, où elle peut causer une morbidité importante, et se situe notamment au niveau du dos des mains et des avant-bras, et de la face antérieure des jambes. On distingue la forme primaire et la forme iatrogène secondaire. La forme primaire est la forme la plus courante, résultant du vieillissement chronologique et d'une exposition prolongée au soleil. Une prédisposition génétique est souvent à l'origine de la forme primaire. La forme iatrogène secondaire est essentiellement une conséquence de traitements chroniques aux corticostéroïdes. L'héliodermie est une autre forme de vieillissement cutané induit par l'exposition prolongée au soleil, se traduisant par un teint jaunâtre et l'apparition de rides profondes, de télangiectases et de lésions pigmentaires, notamment au niveau du dos des mains, des avant-bras, du cou et du décolleté, pouvant entraîner des kératoses actiniques et des mélanomes.

Les manifestations cliniques de l'atrophie cutanée sénile comprennent des marqueurs morphologiques de fragilité (atrophie cutanée, purpura sénile, pseudo-cicatrices stellaires), ainsi que l'expression fonctionnelle de la fragilité cutanée (lacérations cutanées, mauvaise cicatrisation, hémorragies sous-cutanées). Ainsi, dans l'atrophie cutanée sénile, on observe des troubles structurels en relation avec une carence en vitamine C, qu'un traitement par la vitamine C ne permet pas de corriger de manière satisfaisante et reste sans effet visible sur les fibroblastes de l'épiderme.

Au cours du vieillissement cutané, plus particulièrement en cas d'atrophie cutanée sénile, la matrice extracellulaire de la peau perd de sa visco-élasticité. En effet, la matrice extracellulaire contient deux principaux glycosaminoglycanes, le dermatane-sulfate, étroitement lié aux fibres de collagène, et l'acide hyaluronique qui occupe l'espace entre les fibres. Les fibres de collagène sont responsables de l'élasticité de la peau, tandis que l'acide hyaluronique détermine sa viscosité. Ainsi la visco-élasticité de la peau permet d'absorber les contraintes mécaniques qu'elle subit en dissipant l'énergie absorbée tout en reprenant sa structure initiale. Les altérations de la matrice extracellulaire provoquent des fractures des fibres de collagène et une diminution de l'acide hyaluronique, dont la principale conséquence est une perte progressive de sa visco-élasticité.

De nombreuses compositions anti-âge existent mais ne font nullement référence au problème de l'atrophie cutanée sénile, non plus que de l'héliodermie. Certaines compositions sont à base d'acide hyaluronique, incorporé dans des mélanges relativement complexes, et permettent uniquement de lutter contre les signes du vieillissement de la peau. A titre d'exemple, on peut citer le document US-2011/0003020 qui décrit une composition pour application topique permettant de traiter le vieillissement de la peau. Cette composition comprend une quantité pharmaceutiquement efficace d'estradiol (i.e. oestradiol), d'estriol (i.e. oestriol), d'acide hyaluronique et d'un extrait de thé vert, et optionnellement d'une quantité efficace d'acide ascorbique,. FR2939799 décrit des oligosaccharides d'acide glucuronique acétylé comme agents renforçant la cohésion entre le derme et l'épiderme, ainsi améliorant l'élasticité de la peau.

Cependant, malgré les diverses compositions dermatologiques et/ou cosmétiques disponibles, il existe toujours un besoin de pouvoir disposer de nouvelles compositions alternatives ayant de nouveaux effets bénéfiques. A ce titre, une nouvelle composition cosmétique et/ou dermatologique a été découverte par la Demanderesse.

La présente invention a pour objet une composition cosmétique, notamment pour application topique, comprenant de l'acide hyaluronique ou un de ses sels, et de l'acide ascorbique ou un de ses dérivés, lesdits dérivés étant choisis parmi un sel, un ester, un sucre, et un de leurs mélanges, caractérisée en ce que :
- l'acide hyaluronique, ou un de ses sels, a un poids moléculaire d'au plus 1000 kDa, et
- la composition comprend en outre un oligosaccharide autre que l'acide hyaluronique ou un de ses sels, l'oligosaccharide étant un oligosaccharide d'acide glucuronique.

La présente invention a également pour objet une composition pour son application comme médicament dans le traitement de l'atrophie cutanée sénile et/ou de l'héliodermie, et plus particulièrement une composition dermatologique pour son application dans le traitement de l'atrophie cutanée sénile et/ou de l'héliodermie, cette application étant notamment sous forme topique, ladite composition comprenant :
de l'acide hyaluronique ou un de ses sels, et de l'acide ascorbique ou un de ses dérivés, lesdits dérivés étant choisis parmi un sel, un ester, un sucre, et un de leurs mélanges, caractérisée en ce que :
- l'acide hyaluronique, ou un de ses sels, a un poids moléculaire d'au plus 1000 kDa, et
- la composition comprend en outre un oligosaccharide autre que l'acide hyaluronique ou un de ses sels, l'oligosaccharide étant un oligosaccharide d'acide glucuronique.

La composition de l'invention permet avantageusement de stimuler le métabolisme de l'acide hyaluronique passant par une augmentation de l'expression de son récepteur CD44, permettant ainsi de prévenir et/ou de lutter efficacement contre l'atrophie cutanée sénile et l'héliodermie : la visco-élasticité de la peau est donc préservée et/ou améliorée. La présence de l'oligosaccharide permet avantageusement d'optimiser la synergie entre l'acide hyaluronique ou un de ses sels d'une part, et l'acide ascorbique ou un de ses analogues d'autre part.

Cette composition permet de limiter significativement la diminution du taux d'acide hyaluronique dans la peau, voire de l'éviter, et est ainsi garante de l'épaisseur et de la résistance de la peau. Les fonctions des récepteurs CD44 (glycoprotéines transmembranaires polymorphes) ne sont ainsi sensiblement pas affectées et permettent de réguler la prolifération des kératino-cytes en réponse aux stimuli extracellulaires, et de maintenir l'homéostasie de l'acide hyaluronique dans la peau.

### L'acide hyaluronique ou un de ses sels

L'acide hyaluronique est un polymère naturel à base de disaccharide, du type glycosaminoglycane, comprenant des unités acide D-glucuronique et N-acétylglucosamine liées par liaison glucosidique.

Il peut être sous forme libre, hydrolysée, ou bien sous forme d'un de ses sels, tels que des sels de métaux alcalins et alcalino-terreux, par exemple le hyaluronate de sodium, de potassium, de calcium ou de magnésium.

L'acide hyaluronique utilisable dans l'invention est disponible dans le commerce sous diverses formes adaptées selon les utilisations envisagées. Il peut être produit industriellement en quantités importantes par extraction à partir de tissus animaux tels que des crêtes de coq, ou par fermentation bactérienne, ou encore par procédé biotechnologique à partir de substances végétales, par exemple du blé.

Les propriétés de l'acide hyaluronique sont très variables selon son poids moléculaire. L'acide hyaluronique, ou un de ses sels, de poids moléculaire d'au plus 1000 kDa (i.e. faible poids moléculaire) a l'avantage de pouvoir franchir la barrière du *stratum corneum* et ainsi stimuler les récepteurs CD44 responsables de la néosynthèse de l'acide hyaluronique dans le derme.

L'acide hyaluronique, ou un de ses sels, peut avoir un poids moléculaire d'au moins 100 kDa. De préférence, l'acide hyaluronique, ou un de ses sels, peut avoir un poids moléculaire allant de 100 kDa à 800 kDa, et de façon particulièrement préférée un poids moléculaire allant de 200 kDa à 500 kDa.

Les études effectuées par la demanderesse ont montré que le choix d'un acide hyaluronique répondant à cette condition permet une action dans l'épiderme, entraînant une amélioration de la fonction kératinocytaire, sans affecter directement le derme.

La composition de l'invention comprend une quantité efficace d'acide hyaluronique ou un de ses sels, la quantité efficace étant celle qui est nécessaire pour obtenir les effets attendus selon l'invention.

A titre d'exemple, cette quantité représente de 0,01 % à 5% du poids total de la composition, et de préférence de 0,1% à 1% du poids total de la composition.

### L'acide ascorbique ou un de ses analogues

L'acide ascorbique (ou vitamine C) est un acide organique, généralement sous forme L (i.e. acide L-ascorbique), car il est habituellement extrait de produits naturels tels que les citrons, les oranges, etc.

Les analogues de l'acide ascorbique sont des dérivés de l'acide ascorbique. Les dérivés de l'acide ascorbique sont choisis parmi des sels, tels que notamment l'ascorbate de sodium, l'ascorbylphosphate de magnésium ou de sodium ; des esters, tels que notamment des esters acétique, propionique ou palmitique ; des sucres, tels que notamment l'acide ascorbique glycosylé ; et un de leur mélange.

L'acide ascorbique, ou un de ses analogues, est de préférence sous une forme stabilisée. En d'autres termes, l'acide ascorbique, ou un de ses analogues, est sous une forme sensiblement non oxydable, et notamment une forme véhiculable par un lipide.

A titre d'exemple d'acide ascorbique stabilisé, on peut citer l'acide éthyl-ascorbique.

La composition de l'invention comprend une quantité efficace d'acide ascorbique ou un de ses analogues, la quantité efficace étant celle qui est nécessaire pour obtenir les effets attendus selon l'invention.

A titre d'exemple, cette quantité représente de 0,01 % à 5 % du poids total de la composition, et de préférence de 0,1 % à 2 % du poids total de la composition.

### L'oligosaccharide

L'oligosaccharide est un oligomère obtenu à partir d'au moins deux oses (i.e. monosaccharides), et de préférence à partir d'au moins quatre monosaccharides, par liaison glycosidique alpha ou béta. Il peut être linéaire, ramifié ou cyclique.

Le nombre maximal de monosaccharides constituant l'oligosaccharide peut aller jusqu'à 30 monosaccharides, et de préférence jusqu'à 24 monosaccharides.

L'oligosaccharide de l'invention fait partie de la famille des oligosaccharides d'acide glucuronique, pouvant être acétylés ou non. Le degré d'acétylation de ces oligosaccharides peut être compris entre 5 et 15%, et de préférence entre 8 et 10%.

L'oligosaccharide de l'invention peut être encapsulé dans des liposomes, par des techniques bien connues de l'homme du métier.

La composition de l'invention comprend une quantité efficace d'oligosaccharide, la quantité efficace étant celle qui est nécessaire pour obtenir les effets attendus selon l'invention.

A titre d'exemple, cette quantité représente de 0,01 % à 5 % du poids total de la composition, et de préférence moins de 1% du poids total de la composition.

Dans un mode de réalisation particulier, la composition selon l'invention peut comprendre en outre un vecteur de principe actif, dans lequel sont encapsulés l'acide hyaluronique ou un de ses sels ; l'acide ascorbique ou un de ses analogues ; et optionnellement l'oligosaccharide autre que l'acide hyaluronique ou un de ses sels.

A titre d'exemple de vecteur de principe actif, on peut citer la référence X50, commercialisée par la société Infinitec, constitué d'une capsule à double couche associée à un ligand peptidique, par exemple un palmitoyl heptapeptide, permettant une libération contrôlée des principes actifs. Les essais effectués ont montré que ce vecteur permet de libérer les principes actifs de la composition de l'invention, c'est-à-dire l'acide hyaluronique de faible poids moléculaire, l'acide ascorbique et l'oligosaccharide, au niveau des fibroblastes, sur la paroi desquels ils se fixent préférentiellement.

L'encapsulation de ces trois principes actifs dans ledit vecteur peut être réalisée par tous types de techniques bien connues de l'homme du métier.

Les compositions conformes à la présente invention peuvent être présentées sous les formes classiquement utilisées pour une application topique, c'est-à-dire sous forme de gel, lotion, émulsion (en particulier crème ou lait), spot-on, sérum (ou essence), masque ou pommade, contenant des excipients et supports usuels compatibles et pharmaceutiquement acceptables. Elles peuvent aussi se présenter sous forme de patches, tulles ou pansements à libération contrôlée, ou de lingettes imbibées d'une composition selon l'invention.

Ces formes d'administration par voie topique sont préparées par des techniques bien connues de l'homme du métier, et par exemple, dans le cas d'une crème, par dispersion d'une phase grasse dans une phase aqueuse pour obtenir une émulsion huile dans eau, ou inversement pour préparer une émulsion eau dans huile.

La composition selon l'invention peut comprendre un ou plusieurs excipients usuels, adaptés notamment à une administration topique externe, en particulier des excipients acceptables sur le plan dermatologique et cosmétologique.

Ces excipients appropriés pour la formulation sont bien connus de l'homme du métier, et peuvent être choisis par exemple parmi un ou plusieurs des composés i à xv listés ci-dessous :
i. des vitamines antioxydantes, différentes de la vitamine C ou un de ses analogues, telles que la vitamine E, par exemple l'acétate de tocophérol ou le tocotriénol ; les polyphénols naturels ;
ii. des vecteurs de principes actifs, tels que des cyclodextrines ; des liposomes, par exemple de la lécithine ;
iii. des huiles végétales telles que par exemple de l'huile de Kukui, de l'huile de Calophyllum Inophyllum, de l'huile de Helichrysum, de l'huile de Macadamia, de l'huile d'Inca inchi, de l'huile de figue de barbarie, de l'huile d'argan ;
iv. des gélifiants et/ou épaississants, tels que des gommes naturelles comme la gomme xanthane ; des polymères de synthèse, par exemple des polyacrylamides du type Carbopol, des acrylates réticulés et des dérivés de cellulose ;
v. des émulsionnants et co-émulsionnants, tels que arachidyl glucoside ; cétéaryl glucoside ; behenyl alcohol ; cétéaryl alcohol ; céto-stéarylique alcohol ; cetyl alcohol, steareth-2 ; steareth-21 ; isostearyl isostearate ; myristate d'isopropyle, esters de sorbitans et polysorbates ;
vi. des émollients et des tensioactifs, tels que octyl dodecanol ; isononyl isononanoate ; capric/caprylic triglycérides ; l'octanoate de cétéaryle ; le myristate d'isopropyle ; l'isononanoate de cétéaryle ; le 3-diisostéarate de polyglycéryle ; le polyisobutène hydrogéné ; le palmitate cétylique ; le phosphate cétylique ; des huiles végétales telles que l'huile de macadamia ;
vii. des silicones tels que dimethicone, cyclomethicone ;
viii. des humectants / des agents hydratants, tels que la glycérine ; le butylène glycol ; le propylène glycol ;
ix. des conservateurs, tels que le phénoxyéthanol, l'acide déhydro-acétique ; l'acide benzoïque ; le sorbate de potassium ; le benzoate de sodium ; des parahydroxybenzoates de méthyle ou d'éthyle ;
x. des agents de protection contre les rayons ultraviolets, tels que des filtres solaires UV-A et UV-B hydrophiles ou lipophiles, choisis parmi la benzophénone ou un dérivé de benzophénone tel que la 2-hydroxy-4-méthoxy-benzophénone ; un ester d'acide cinnamique, et plus particulièrement le méthoxycinnamate d'octyle et le méthoxycinnamate d'éthyl-2-hexyle, ou encore un cyano-β, β-diphénylacrylate tel que l'octo-crylène, le 4-méthylbenzylidène camphre, et des dérivés du dibenzoylméthane tels que le 4-isopropyl dibenzoylméthane, le t-butyl-méthoxy dibenzoylméthane, et le 4-méthoxy-dibenzoylméthane ; ou tels que des pigments formant un écran UV ;
xi. des poudres exfoliantes ; des poudres de fruits ; ou des poudres matifiantes telles que le copolymère de méthacrylate de méthyle ;
xii. des agents neutralisants ;
xiii. des solubilisants ;
xiv. des colorants ;
xv. des parfums.

La composition selon l'invention peut comprendre de façon particulièrement préférée au moins un émollient, tel que ceux cités précédemment (voir le point vi ci-avant). Elle peut comprendre entre 10 et 60 % en poids d'émollient(s) selon le type de formulation envisagée.

La composition selon l'invention peut en outre comprendre un ou plusieurs actifs complémentaires usuels, adaptés notamment à une administration topique externe, en particulier des actifs acceptables sur le plan dermatologique et cosmétologique.

Ces actifs complémentaires appropriés pour la formulation sont bien connus de l'homme du métier, et peuvent être par exemple des actifs anti-âges, des actifs apaisants, des actifs purifiants, des actifs hydratants, etc.

Un autre objet de l'invention est une utilisation de la composition cosmétique et/ou dermatologique telle que décrite dans la présente invention, pour lutter contre les signes du vieillissement cutané.

D'autres caractéristiques et avantages de la présente invention apparaîtront à la lumière des exemples qui vont suivre, lesdits exemples étant donnés à titre illustratif et nullement limitatif.

### Exemple de formulation d'un gel

Suivant les techniques classiques, on prépare un gel à base d'une composition selon l'invention ayant la composition pondérale suivante :

**Tableau 1**

| | Emulsion eau dans huile |
|---|---|
| Acide hyaluronique | 0,05 |
| Acide ascorbique | 1,0 |
| Oligosaccharide | 1,0 |
| Emollient | 20 |
| Eau q.s.p. | 100 |

L'origine des constituants de l'émulsion eau-dans-huile du tableau 1 est la suivante :
- Acide hyaluronique est un acide hyaluronique de 150 kDa, commercialisé par la société Contipro, sous la référence HySilk ;
- Acide ascorbique est de la vitamine C stabilisée sous forme d'acide éthyl-ascorbique, commercialisée par la société Grant sous la référence Corum 9515 ;
- Oligosaccharide est la formulation commercialisée par la société Sederma, sous la référence Subliskin, cette formulation comprenant des oligosaccharides d'acide glucuronique acétylé (nombre de monosaccharides compris entre 12 et 24, et degré d'acétylation compris entre 8 et 10%) dans des liposomes ; et
- Emollient est un émollient classique tel que par exemple un de ceux cités dans la présente invention.

Ce gel peut être facilement appliqué notamment au niveau du décolleté, du dos des mains, des avant-bras, et/ou de la face antérieure des jambes.

Pour traiter l'atrophie cutanée sénile, ce gel peut être appliqué deux fois par jour, de préférence matin et soir, pendant au moins 3 semaines, jusqu'à obtention de résultats satisfaisants.

Plus généralement, pour traiter et prévenir des signes du vieillissement cutané, le gel décrit ci-avant peut être appliqué au moins une fois par jour, de préférence le soir, pendant au moins deux mois.

Des études cliniques sur des patients souffrant d'atrophie cutanée sénile à un stade plus ou moins avancé (stades 1 à 4) ont mis en évidence une action efficace de l'association acide hyaluronique et de la vitamine C, selon la présente invention, pour traiter l'atrophie cutanée sénile.

Les stades 1 à 4 peuvent être définis comme suit :
- Stade 1 : ce stade est caractérisé par un fort amincissement de la peau avec purpura sénile et pseudo-cicatrices stellaires ; l'évaluation clinique de la visco-élasticité de la peau est un bon paramètre de mesure ;
- Stade 2 : en plus des lésions observées dans le stade 1, on observe de petites lacérations cutanées localisées, lesquelles résultent d'un clivage entre le derme et l'épiderme ;
- Stade 3 : les lacérations cutanées sont plus nombreuses et plus grandes, et peuvent impliquer toute la surface des extrémités ; il y a clairement un retard de cicatrisation ;
- Stade 4 : la progression de ces lésions conduit à l'apparition d'hématomes cutanés disséquants, constituant une urgence médicale et nécessitant une hospitalisation.

Une étude comparative a été réalisée par la Demanderesse entre d'une part une composition selon l'invention (produit A) comprenant de l'acide hyaluronique d'un poids moléculaire compris entre 100 et 500 kDa et de la vitamine C, et d'autre part ladite vitamine C seule (produit B).

Après avoir réalisé des biopsies cutanées chez une femme de 93 ans au niveau d'un fond de ride (fibroblastes de fond de rides : FR) et de la peau avoisinante (fibroblastes « sains » : FS), ces biopsies ont été mises en culture par des procédés bien connus de l'homme du métier.

A partir de ces biopsies mises en culture, différents tests ont été réalisés en présence du produit A et du produit B, à savoir :
- test sur la migration cellulaire pendant 15 jours ;
- test sur la synthèse de collagène I en prélevant des surnageants de culture après 0, 4, 21 et 42 jours de culture, puis en dosant le collagène I par la méthode Elisa ;
- test sur la production de lactates en prélevant des surnageants de culture après 0, 4, 21 et 42 jours de culture, puis en dosant les lactates par spectrophotométrie ; et
- test sur la peroxydation lipidique.

Les résultats sont les suivants :
- sur la migration cellulaire : le produit A ne perturbe pas la migration des fibroblastes issus de fond de rides par rapport au produit B (produit témoin) ;
- sur la synthèse de collagène : effet bénéfique à long terme (i.e. après au moins 8 jours, et de préférence après 21 jours de culture) du produit A sur la production de collagène I des fibroblastes issus de fond de rides, contrairement au produit B pour lequel la synthèse de collagène est moindre ;
- sur la production de lactates : la production de lactates en présence du produit A est augmentée ce qui témoigne de l'activité métabolique bénéfique des cellules issues de fond de rides ;
- sur la peroxydation lipidique : la peroxydation lipidique est diminuée en présence du produit A par rapport au produit B.

Par conséquent, le produit A restaure l'activité de synthèse (collagène) et métabolique des fibroblastes tout en ayant un pouvoir antioxydant protecteur, contrairement au produit B qui a un effet beaucoup moins significatif sur ce type d'activité ainsi qu'un effet pro-oxydant sensiblement délétère.

Ces tests ont permis de montrer clairement un effet bénéfique significatif du produit A sur les fibroblastes de fond de rides, et notamment sur ses propriétés anti-héliodermiques et pour traiter et prévenir des signes du vieillissement cutané.

## Revendications

1. Composition cosmétique formulée pour une application topique, comprenant de l'acide hyaluronique ou un de ses sels, et de l'acide ascorbique ou un de ses dérivés, lesdits dérivés étant choisis parmi un sel, un ester, un sucre, et un de leurs mélanges, **caractérisée en ce que** :
- l'acide hyaluronique, ou un de ses sels, a un poids moléculaire d'au plus 1000 kDa, et
- la composition comprend en outre un oligosaccharide autre que l'acide hyaluronique ou un de ses sels, l'oligosaccharide étant un oligosaccharide d'acide glucuronique.

2. Composition pour son application comme médicament dans le traitement de l'atrophie cutanée sénile et/ou de l'héliodermie, ladite composition comprenant :
- de l'acide hyaluronique, ou un de ses sels, ayant un poids moléculaire d'au plus 1000 kDa,
- de l'acide ascorbique ou un de ses dérivés, lesdits dérivés étant choisis parmi un sel, un ester, un sucre, et un de leurs mélanges, et
- un oligosaccharide autre que l'acide hyaluronique ou un de ses sels, l'oligosaccharide étant un oligosaccharide d'acide glucuronique,
la composition étant formulée pour une application topique.

3. Composition selon l'une quelconque des revendications précédentes, **caractérisée en ce que** l'acide hyaluronique, ou un de ses sels, a un poids moléculaire d'au moins 100 kDa, et de préférence d'au moins 200 kDa.

4. Composition selon l'une quelconque des revendications précédentes, **caractérisée en ce que** l'acide hyaluronique, ou un de ses sels, a un poids moléculaire allant de 200 kDa à 500 kDa.

5. Composition selon l'une quelconque des revendications précédentes, **caractérisée en ce que** l'acide ascorbique, ou un de ses dérivés, est sous une forme stabilisée.

6. Composition selon l'une quelconque des revendications précédentes, **caractérisée en ce que** l'oligosaccharide est obtenu à partir d'au moins quatre monosaccharides.

7. Composition selon l'une quelconque des revendications précédentes, **caractérisée en ce qu'**elle comprend de 0,01 % à 5 % en poids d'acide ascorbique ou un de ses dérivés, par rapport au poids total de la composition.

8. Composition selon l'une quelconque des revendications précédentes, **caractérisée en ce qu'**elle comprend de 0,01 % à 5 % en poids d'acide hyaluronique ou un de ses sels, par rapport au poids total de la composition.

9. Composition selon l'une quelconque des revendications précédentes, **caractérisée en ce qu'**elle comprend de 0,01 % à 5 % en poids d'oligosaccharide autre que l'acide hyaluronique ou un de ses sels, par rapport au poids total de la composition.

10. Composition selon l'une quelconque des revendications précédentes, **caractérisée en ce qu'**elle comprend en outre un peptide en tant que vecteur de principe actif, dans lequel sont encapsulés :
- l'acide hyaluronique ou un de ses sels ;
- l'acide ascorbique ou un de ses dérivés ; et
- optionnellement l'oligosaccharide autre que l'acide hyaluronique ou un de ses sels.

11. Composition selon l'une quelconque des revendications précédentes, **caractérisée en ce qu'**elle comprend en outre au moins un émollient.

12. Composition selon l'une quelconque des revendications précédentes, **caractérisée en ce que** les dérivés de l'acide ascorbique sont choisis parmi l'ascorbate de sodium, l'ascorbylphosphate de magnésium ou de sodium ; des esters acétique, propionique ou palmitique ; l'acide ascorbique glycosylé ; et un de leur mélange.

13. Utilisation non thérapeutique de la composition selon l'une quelconque des revendications 1, 3 à 12, pour lutter contre l'atrophie cutanée sénile.

14. Utilisation non thérapeutique de la composition selon l'une quelconque des revendications 1, 3 à 12, pour lutter contre l'héliodermie.

## Patentansprüche

1. Kosmetische Zusammensetzung, die für eine topische Anwendung formuliert ist, umfassend Hyaluronsäure oder eines ihrer Salze und Ascorbinsäure oder eines ihrer Derivate, wobei die Derivate aus einem Salz, einem Ester, einem Zucker und einem ihrer Gemische ausgewählt sind, **dadurch gekennzeichnet, dass**:
- die Hyaluronsäure oder eines ihrer Salze ein Molekulargewicht von höchstens 1000 kDa hat, und
- die Zusammensetzung ferner ein anderes Oligosaccharid als die Hyaluronsäure oder eines ihrer Salze umfasst, wobei das Oligosaccharid ein Oligosaccharid der Glucuronsäure ist.

2. Zusammensetzung für ihre Anwendung als Arzneimittel bei der Behandlung der senilen Hautatrophie und/oder der Lichtalterung, wobei die Zusammensetzung umfasst:
- Hyaluronsäure oder eines ihrer Salze mit einem Molekulargewicht von höchstens 1000 kDa,
- Ascorbinsäure oder eines ihrer Derivate, wobei die Derivate aus einem Salz, einem Ester, einem Zucker und einem ihrer Gemische ausgewählt sind, und
- ein anderes Oligosaccharid als die Hyaluronsäure oder eines ihrer Salze, wobei das Oligosaccharid ein Oligosaccharid der Glucuronsäure ist,
wobei die Zusammensetzung für eine topische Anwendung formuliert ist.

3. Zusammensetzung nach einem der vorangehenden Ansprüche, **dadurch gekennzeichnet, dass** die Hyaluronsäure oder eines ihrer Salze ein Molekulargewicht von mindestens 100 kDa und vorzugsweise von mindestens 200 kDa hat.

4. Zusammensetzung nach einem der vorangehenden Ansprüche, **dadurch gekennzeichnet, dass** die Hyaluronsäure oder eines ihrer Salze ein Molekulargewicht von 200 kDa bis 500 kDa hat.

5. Zusammensetzung nach einem der vorangehenden Ansprüche, **dadurch gekennzeichnet, dass** die Ascorbinsäure oder eines ihrer Derivate in einer stabilisierten Form vorliegt.

6. Zusammensetzung nach einem der vorangehenden Ansprüche, **dadurch gekennzeichnet, dass** das Oligosaccharid aus mindestens vier Monosacchariden gewonnen ist.

7. Zusammensetzung nach einem der vorangehenden Ansprüche, **dadurch gekennzeichnet, dass** sie 0,01 bis 5 Gew.-% Ascorbinsäure oder eines ihrer Derivate in Bezug auf das Gesamtgewicht der Zusammensetzung umfasst.

8. Zusammensetzung nach einem der vorangehenden Ansprüche, **dadurch gekennzeichnet, dass** sie 0,01 bis 5 Gew.-% Hyaluronsäure oder eines ihrer Salze in Bezug auf das Gesamtgewicht der Zusammensetzung umfasst.

9. Zusammensetzung nach einem der vorangehenden Ansprüche, **dadurch gekennzeichnet, dass** sie 0,01 bis 5 Gew.-% eines anderen Oligosaccharids als die Hyaluronsäure oder eines ihrer Salze in Bezug auf das Gesamtgewicht der Zusammensetzung umfasst.

10. Zusammensetzung nach einem der vorangehenden Ansprüche, **dadurch gekennzeichnet, dass** sie ferner ein Peptid als Vektor des aktiven Prinzips umfasst, in welches eingekapselt sind:
- die Hyaluronsäure oder eines ihrer Salze;
- die Ascorbinsäure oder eines ihrer Derivate; und
- optional das andere Oligosaccharid als die Hyaluronsäure oder eines ihrer Salze.

11. Zusammensetzung nach einem der vorangehenden Ansprüche, **dadurch gekennzeichnet, dass** sie ferner mindestens einen Weichmacher umfasst.

12. Zusammensetzung nach einem der vorangehenden Ansprüche, **dadurch gekennzeichnet, dass** die Derivate der Ascorbinsäure aus dem Natriumascorbat, dem Magnesium- oder Natriumascorbylphosphat; dem Essigsäure-, Propion- oder Palmitinester; der glycolysierten Ascorbinsäure und einem ihrer Gemische ausgewählt sind.

13. Nicht therapeutische Verwendung der Zusammensetzung nach einem der Ansprüche 1, 3 bis 12 zur Bekämpfung der senilen Hautatrophie.

14. Nicht therapeutische Verwendung der Zusammensetzung nach einem der Ansprüche 1, 3 bis 12 zur Bekämpfung der Lichtalterung.

## Claims

1. Cosmetic composition formulated for topical application, comprising hyaluronic acid or one of the salts thereof, and ascorbic acid or one of the derivatives thereof, said derivatives being selected from among a salt, ester, sugar and one of the mixtures thereof, **characterized in that**:
- the hyaluronic acid or one of the salts thereof has a molecular weight of no more than 1000 kDa; and
- the composition further comprises an oligosaccharide other than hyaluronic acid or salt thereof, the oligosaccharide being an oligosaccharide of glucuronic acid.

2. Composition for application thereof as medicinal product in the treatment of senile skin trophy and/or photoaging, said composition comprising:
- hyaluronic acid or one of the salts thereof, having a molecular weight of no more than 1000 kDa;
- ascorbic acid or one of the derivatives thereof, said derivatives being selected from among a salt, ester, sugar and one of the mixtures thereof; and
- an oligosaccharide other than hyaluronic acid or one of the salts thereof, the oligosaccharide being an oligosaccharide of glucuronic acid;
the composition being formulated for topical application.

3. The composition according to any of the preceding claims, **characterized in that** the hyaluronic acid, or one of the salts thereof, has a molecular weight of at least 100 kDa, and preferably of at least 200 kDa.

4. The composition according to any of the preceding claims, **characterized in that** the hyaluronic acid or one of the salts thereof has a molecular weight ranging from 200 kDa to 500 kDa.

5. The composition according to any of the preceding claims, **characterized in that** the ascorbic acid or one of the derivatives thereof is in a stabilised form.

6. The composition according to any of the preceding claims, **characterized in that** the oligosaccharide is obtained from at least four monosaccharides.

7. The composition according to any of the preceding claims, **characterized in that** it comprises from 0.01 to 5 weight % of ascorbic acid or one of the derivatives thereof, relative to the total weight of the composition.

8. The composition according to any of the preceding claims, **characterized in that** it comprises from 0.01 to 5 weight % of hyaluronic acid or one of the salts thereof, relative to the total weight of the composition.

9. The composition according to any of the preceding claims, **characterized in that** it comprises from 0.01 to 5 weight % of oligosaccharide other than hyaluronic acid or one of the salts thereof, relative to the total weight of the composition.

10. The composition according to any of the preceding claims **characterized in that**, as active ingredient vector, it further comprises a peptide which encapsulates:
- the hyaluronic acid or one of the salts thereof;
- the ascorbic acid or one the derivatives thereof; and
- optionally, the oligosaccharide other than hyaluronic acid or one of the salts thereof.

11. The composition according to any of the preceding claims, **characterized in that** it also comprises at least one emollient.

12. The composition according to any of the preceding claims, **characterized in that** the derivatives of ascorbic acid are selected from among sodium ascorbate, magnesium or sodium ascorbyl phosphate; acetic, propionic or palmitic esters; glycosylated ascorbic acid; and one of the mixtures thereof.

13. Non-therapeutic use of the composition according to any of claims 1, 3 to 12, to combat senile skin atrophy.

14. Non-therapeutic use of the composition according to any of claims 1, 3 to 12 to combat photoaging.
